# EUROPEAN PATENT SPECIFICATION

(11) **EP 0 365 646 B1**
(45) Date of publication and mention of the grant of the patent: **08.05.1996**
(21) Application number: 89904898.7
(22) Date of filing: 13.03.1989
(51) Int. Cl.: A61K 39/02, C07K 7/06, C07K 7/08, C07K 14/195

(54) **SYNTHETIC PEPTIDES FROM STREPTOCOCCAL M PROTEIN AND VACCINES PREPARED THEREFROM**
VOM STREPTOCOCCUS M-PROTEIN ABGELEITETE SYNTHETISCHE PEPTIDE UND DAMIT HERGESTELLTE IMPFSTOFFE
PEPTIDES SYNTHETIQUES PROVENANT DE PROTEINES STREPTOCOCCIQUES M ET VACCINS PREPARES A PARTIR DE CES PEPTIDES

(30) Priority: 25.03.1988 US 173380; 27.02.1989 US 315588
(43) Date of publication of application: 02.05.1990
(73) Proprietor: THE ROCKEFELLER UNIVERSITY, New York, NY 10021 (US)
(72) Inventor: FISCHETTI, Vincent, A., West Hempstead, NY 11552 (US)
(74) Representative: Weinhold, Peter, Dr.
(86) International application number: US8901026
(87) International publication number: WO8909064

(56) References cited:
- US-A- 4 705 684
- THE JOURNAL OF IMMUNOLOGY, vol. 141, no. 10, 1988, pages 3592-2599, The American Association of Immunologists, US; V.A. FISCHETTI et al.: "Mapping the immunodeterminants of the complete streptococcal M6 protein molecule"
- J. EXP. MED., vol. 164, 1986, pages 1226-1238, The Rockefeller University
- Press; K.F. JONES et al.: "Immunochemical localization and amino acid sequences of crossreactive epitopes within the group A streptococcal M6 protein"
- J. EXP. MED., vol. 167, 1988, pages 1114-1123, The Rockefeller Universtiy Press; K.F. JONES et al.: "The importance of the location of antibody binding on the M6 protein for opsonization and phagocytosis of group A M6 streptococci"
- The Journal of Biological Chemistry, Volume 261, No. 4, issued 05 February 1986, page 1677-1686, HOLLINGSHEAD et al, "Complete Nucleotide Sequence of Type 6 M Protein of the Group A Streptococcus: Repetitive Structure and Membrane Anchor" see page 1677-1686, particularly figure 2, sequence 216-235, 248-269 and 275-284.
- Infection and Immunity, Volume 56, No. 10, issued October 1988, page 2666-2672, BESSEN et al, "Influence of Intranasal Immunization with Synthetic Peptides Corresponding to Conserved Epitopes of M Protein on Mucosal Colonization By Group A Streptococci "see page 2666-2671.
- Journal of Immunology, Volume 139, No. 4, issued 15 August 1987, page 1285- 1290, SARGENT et al, "Sequence of Protective Epitopes of Streptococcal M
- Proteins shared with Cardiac Sarcolemmal Membranes" see pages 1288-1289.
- Proceedings National Academy of Science USA, Volume 82, pages 1822-1826, SCOTT et al, "Relationship of M Protein Genes in Group A Streptococci" see pages 1822-1826. March 1985.

## Description

The M protein of group A streptococci is a fibrous dimer of helices arranged in a coiled coil extending about 50 nm from the surface of these organisms. It is a fibrillar molecule of which there exist more than 80 serological types. M protein renders the streptococcus resistant to nonimmune phagocytosis. It is the major virulence factor of streptococcal bacteria.

Hollingshead et al. (The Journal of Biological Chemistry, 261, 1677-1686; 1986) disclose the DNA sequence of the gene for type 6M protein of Streptococcus pyogenes. Based on antibody cross-reactivity tests and on DNA hybridisation experiments, the authors suggest the possibility of regions in the M6 protein common to all M types of group A streptococci, which might be useful for development of a peptide vaccine. However, they do not indicate the location or sequence of suitable peptides.

Fig. 1 shows the complete amino acid sequence of the M6 protein from strain D471 of a group A streptococcal culture from The Rockefeller University collection.

Fig. 2 illustrates a model of the complete M6 protein from strain D471 as it exists on the cell wall.

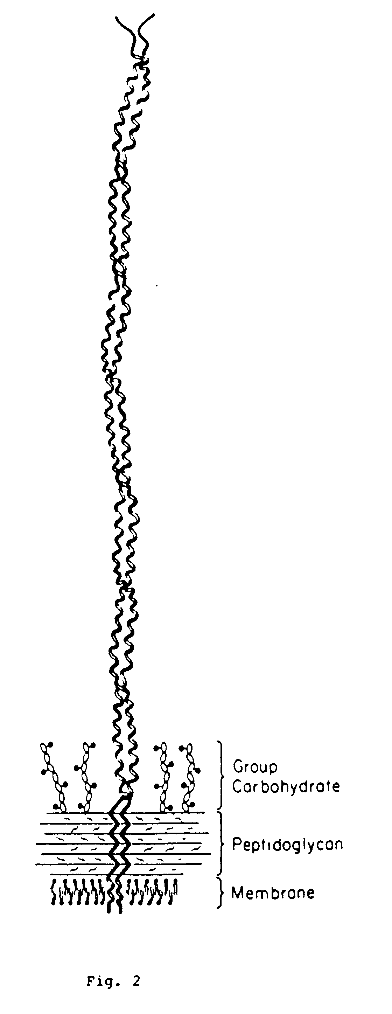

It will be seen from Fig. 2 that a portion of the M protein, the carboxy terminus, is embeded in the peptidoglycan and membrane of the cell wall. The adjacent segment is sheltered by the carbohydrate of the cell wall which is composed of a rhamnose backbone (open circle) and N-acetylglycosamine branches (closed circles). The distal amino terminus of the M-protein is non-helical. There is an exposed region between the carbohydrate shelter and the non-helical region.

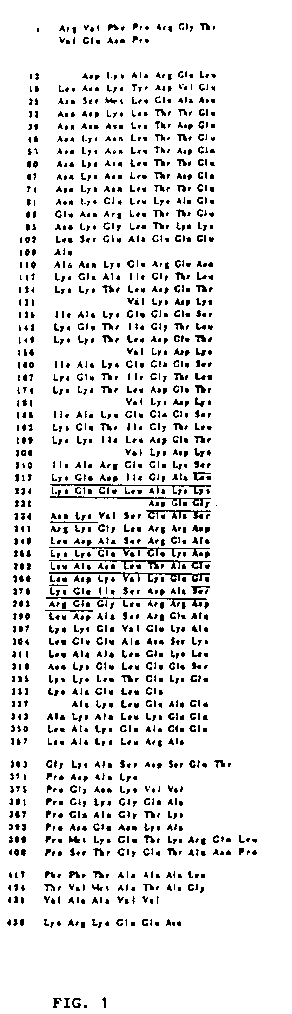

Fig. 1 is the complete amino acid sequence of an M protein from a specific strain. M proteins from other strains will have generally the same structural features and conformation, but the amino acid sequences will vary. The principal variations occur towards the amino terminal. The molecules become more and more conserved towards the carboxy end. Thus homology within M molecules of different serotypes progressively increases at sites which are closer to the carboxy terminus and more proximal to the cell wall.

It is the variability at the amino terminus which is responsible for the antigenic variation of the M protein. Antibodies which afford protection against one serotype are not effective to resist infection by other serotypes in an opsonophagocytosis assay. Thus, it is theoretically possible for an individual to be infected many times with different streptococcal strains, and each infection will continue until the immune system has generated a sufficient concentration of antibodies to neutralize the specific infecting strain.

Experience shows, however, that streptococcal infections are almost exclusively limited to children, peaking at age 7. Adults are apparently resistant to such infections, presumeably because they have built up an immunity which is effective against most serotypes. Hence, there may be an immunological response to streptococcal infection which produces memory type antibodies which recognize epitopes on most, if not all streptococcus serotypes. It has now been discovered that these epitopes are in the conserved region of the M protein which is not protected by its proximity to the cell wall, i.e., the conserved, exposed region of the M protein. While the identity of the amino acids in this region varies somewhat amongst serotypes, it generally runs from about position 170 to position 296 on the M protein molecule.

Polypeptides from that region are capable of eliciting a protective immune response when administered to a mammal in need of protection against streptococcal infection.

As is known, the mammalian body has several methods for protecting itself against infection by microorganisms. One is the adaptive system in which the immune response to an invading organism is the production of IgG antibodies followed by opsonization and phagocytosis mediated by complement. Another is the production or activation of IgA which is the predominant immunoglogulin of seromucous secretions such as saliva, tracheobronchial secretions, colostrum, milk and genito-urinary secretions. Secretory IgA (sIgA) is a form of IgA protected from proteolysis by the secretory component. IgA prevents infective microorganisms from adhering, colonizing and invading the mucous tissue.

The presently preferred procedure for the practice of this invention involves principally the stimulation of sIgA by intranasal or oral administration. There may be concomitant production of IgG, and both may contribute to the immunization. The invention will be described principally as applied to that procedure. The polypeptides of the invention may also be used to stimulate IgG as the principal response by parenteral administration with concomitant production of low level IgA.

The polypeptides used in the invention are selected from polypeptides in the conserved exposed section of the M protein. Generally they will contain at least 5 amino acid segments and will be administered as haptens conjugated to a carrier. Generally, it is impractical for the polypeptide to contain more than 25 amino acid segments because the synthesis of pure material becomes more difficult as the number of amino acid residues in the peptide increases.

The polypeptide can be obtained by selective enzymatic or chemical cleavage from the M protein, but it is far more preferable to synthesize the selected polypeptide using any of the known techniques, e.g., the solid phase Merrifield synthesis where the peptide is synthesized on a resin substrate, separated and purified. Utilizing this procedure polypeptides having the exact sequence of amino acids from the selected site on the conserved exposed section of the M protein can be produced. However, it is not essential that an exact sequence be employed. Minor modifications, especially those that do not change the conformation of the peptide can be made by substituting one or more amino acids to produce useful polypeptides having substantially the same sequence as in the M protein. However, the sequence of the polypeptide will usually have the same sequence as in the natural product.

It may also be desirable to add one or several amino acids to either terminus of the selected polypeptide. This variation of the invention will be typically employed to bind the polypeptide to a carrier or to increase its immunogenicity.

The polypeptides of this invention are:
(1) Ser-Lys-Gln-Asp-Ile-Gly-Ala-Leu-Lys-Gln-Glu-Leu-Ala-Lys-Lys-Asp-Glu-Gly-Asn-Lys
(2) Leu-Asp-Ala-Ser-Arg-Glu-Ala-Lys-Lys-Gln-Val-Glu-Lys-Asp-Leu-Ala-Asn-Leu-Thr-Ala-Glu-Leu
(3) Glu-Lys-Gln-Ile-Ser-Asp-Ala-Ser-Arg-Gln
The first peptide will sometimes hereinafter be referred to as peptide 216-235, the second as 248-269, the third as 275-284. The numerals refer to the position of the first and the last amino acid segment in the polypeptide on the M protein molecule, see Fig. 1.

The epitopes represented by these peptides are present in the conserved exposed region of the majority of the known serotypes present in nature. Therefore, if they are shown to raise antibodies against one serotype, they would be expected to have the same response with other serotypes.

These peptides were synthesized with an additional cysteine residue on the carboxy terminus by the method of Barany and Merrifield. See The Peptides: Analysis, Synthesis; Biology. E. Gross and J. Meienhofer, editors. Academic Press, Inc. New York, 1-284. They were purified by reverse phase liquid chromatography on a Brownlee C-8 column, eluted as as single peak using a gradient of acetonitrile in 0.1% trifluoroacetic acid and stored in lyophilized form at 4^{o}C. The amino acid sequence was verified both by amino acid composition and sequence determination to the penultimate residue. To eliminate disulfide bridges formed upon storage, several days prior to conjugation to cholera toxin B subunit (CBT) the peptides were reduced with 0.14 M beta-2-mercaptoethanol at pH 7.2 and subjected to several cycles of lyophilization and solubilization to eliminate the reducing agent.

These peptides were used to prepare vaccines for both oral and intranasal immunization of mammals. For the preparation the primary amino groups highly purified CTB in phosphate buffered saline (PBS) were derivatized by addition of a 15 molar excess of the heterobifunctional crosslinking agent N'-succinimidyl 3-(-2- pyridyldithio) propionate (SPDP; Pierce Chemical Co., Rockford, IL) solubilized in ethanol. The mixture was continuously stirred with a magnetic stirrer until a precipititate formed (about 10 to 15 min).

At this time, the reaction was stopped by addition of ethanolamine to a final concentration of 70 mM, which resulted in immediate clearance of the precipitate. The solution was dialyzed overnight against PBS at 4^{o}C. A small sample of the dialysate was removed and the absorbance at 343 nm was measured before and after addition of 5mM dithiothreitol to establish the extend of derivatization based on the release of pyridine-2-thione. See Carlson, et al. Biochem. J. 173, 723 (1988). Dialyzed CTB was mixed with a single peptide at a 1:1.5 w/w ratio at room temperature for 4 h, then overnight at 4^{o}C. Each of the three individual peptide-CTB conjugates (containing unbound peptide) were pooled in equal weight quantities and aliquots stored at -80^{o}C until use. Free peptide was not separated from peptide-CTB conjugates, and the entire mixture containing free peptide plus linked peptide-CTB was used for immunization. The average number of peptide molecules covalently linked per CTB monomer was calculated based on A₃₄₃ of the mixture (Table 1). This degree of substitution preserved nearly 100% of the CTB binding capacity to GM₁ as compared to underivatized CTB (data not shown), determined in a GM₁ binding assay (25). See Tsang, et al. Meth Enz. 92,391 (1983). Increased substitution of CTB with SPDP led to a marked decrease in GM₁ binding capacity.

**TABLE 1**

| Covalent Linkage of Peptides to CTB. Molar Ratio | |
|---|---|
| Peptide | Peptide:CTB (a) |
| 216-235 | 1.12 |
| 248-269 | 1.20 |
| 275-284 | 1.14 |

| | |
|---|---|
| (a) Molar ratio peptide to CTB in covalently linked form based on pyridine-2-thione release measured at 343 nm. | |

To determine the ability of the selected peptides and their conjugates to elicit an immunological response in mammals, mice were immunized intranasally (i.n.) 3 times over a 6 day period with the vaccine prepared as described above. Control mice were treated with CTB alone. The animals were rested 3 weeks and boosted i.n. with a single dose of antigen. Each dose contained 20 ug of CTB with or without a total of 12 ug of each peptide. The quantity of peptide indicated represents the total of free and covalently bound product. The vaccine was delivered to the nares of unanesthetized mice (10 ul per nostril) through a Hamilton syringe (model 750) fitted with a repeating dispenser (PB600) and a blunt end needle. Female outbred Swiss CD1 mice (Charles River) were 4 to 5 weeks of age at the onset of immunization.

The same composition may be administered directly in to the oral cavity.

Type 6 streptococci (strain S43/192 from The Rockefeller University collection) were used to challenge the vaccinated mice. The strain was selected for resistance to 200 ug/ml streptomycin. Mouse virulence of S43/192 was maintained by several intraperitoneal passages as described by Phillips, et al. Proc. Natl. Acad. Sci. USA 78, 4689-4693 (1981). A single stock of organisms was prepared from an overnight culture, concentrated 10-fold, frozen at-80^{o}C, and used for all challenge experiments. Stocks were diluted 1:500 and grown overnight at 37^{o}C in Todd-Hewitt broth, then diluted 1:20 in fresh growth medium. When cultures reached an OD₆₅₀ of 0.5 (18 mm tube), they were centrifuged and resuspended in saline to one-sixth the volume (approximately 2.5 x 10⁸ colony forming units per ml). A peptide-CTB vaccinated group of mice was compared to a control group (CTB only) in four separate challenges with live streptococci. In each challenge experiment, the peptide-immunized and control groups contained 12 to 14 mice apiece. At 10 days following boost, mice were administered 10 ul per nostril of the streptococcal suspension. Beginning 24 h after challenge, and at 24 or 48 h intervals thereafter, throats were swabbed (Calgiswab type 4, Spectrum) and cultured on blood agar plates containing 200 ug/ml streptomycin. Cultures were grown overnight at 37^{o}C and scored for the presence of beta-hemolytic streptococci.

The throat cultures taken at 24 or 48 hour intervals and colonization of streptococci was assessed. The results of four separate challenges are summarized in Table 2. Animals which had received the peptide-CTB vaccine displayed a decrease in the incidence of pharyngeal infection (plus mortality) during the 10 days following streptococcal challenge. The difference in colonization between peptideimmunized mice and the control group was significant for five of the six time points at which pharyngeal cultures were taken. Furthermore, at every throat culture analysis in each of the four separate challenge experiments, the number of positive throat cultures among the control group exceeded that of the peptide-CTB immunized group 96% of the time (23/24 analyses).

**TABLE 2**

| Protective Immunity Induced by Conserved Synthetic Peptides | | | |
|---|---|---|---|
| Day Post Challenge | Positive Throat Culture (plus dead)/ Total Swabbed (%) (a) | | P-value (b) |
| | CTB Only | Conserved Peptide-CTB | |
| 1 | 23/52 (44%) | 11/52 (21%) | P < 0.025 * |
| 2 | 23/52 (44) | 11/52 (21) | P < 0.025 * |
| 4 | 22/52 (42) | 14/52 (27) | NS |
| 6 | 31/52 (60) | 18/52 (35) | P < 0.025 * |
| 8 | 33/52 (63) | 22/52 (42) | P < 0.050 * |
| 9 & 10 | 33/52 (63) | 22/52 (42) | P < 0.050 * |

| | | | |
|---|---|---|---|
| (a) Animals were immunized with peptide-CTB conjugates or CTB only, and challenged with live streptococci in four separate experiments. Mice which died during the course of the experiment were scored as positive. The overall mortality rate was 20%, and was not significantly different for the two groups. | | | |
| (b) P values less than 0.05 (*) were considered to be statistically significant (chi-square analysis). NS, not significant. | | | |

The majority of individual mice displayed one of two patterns of pharyngeal colonization. Fifty-seven percent of survivors which had received CTB only, and 76% of peptide-CTB immunized mice, either remained completely free of streptococci at each throat culture, or carried 25 or more colony forming units for nearly all cultures. The remainder of the survivors typically had positive throat cultures at one or two time points only, and 77% of these cultures showed fewer than 10 colonies. Thus, the method for analyzing pharyngeal infection is highly reproducible, and most animals were in either a stable state of streptococcal carriage or organism-free. It is apparent thus that the polypeptides of this invention are capable of eliciting a protective response to streptococcal colonization in mammals by intranasal administration of an effective dose. The individual antigens may be similarly employed. The actual dosage may vary somewhat, but will generally be of the same order of magnitude as similar vaccines. Similar results are achieved by other methods of administering the vaccine, e.g., oral administration.

Vaccines for use to protect against streptococcal infection have been proposed previously. These vaccines have been prepared from polypeptides from the hypervariable amino end of the molecule. They have emjoyed some success in providing type specific immunity against homologous serotypes. Their performance has been improved by incorporating several type specific determinants in the same multivalent vaccine. However, give the enormous array of existing M serotypes, this procedure is not an attractive one.

The carrier described above is CTB. Those skilled in the art will recognize that other carriers can be employed. These include, for example, the E. coli labile toxin B subunit or the pili from E. coli cells identified as K99 pili and 987P pili described by Aizpurua and Russell-Jones in J. Exp. Med. 167, 440 (1988). The antigen need not be actually joined to the carrier. The two may be coadministered to achieve substantially the same effect.

Other natural carriers which can be employed in the practice of this invention, especially for parenteral administration include tetanus toxoid, keyhole limpet hemocyanin, bovine serum albumin or ovalbumin. Synthetic carriers are also known and can be employed.

The vaccines of this invention may be administered parenterally in an emulsion with various adjuvants. The adjuvants aid in attaining a more durable and higher level of immunity using smaller amounts of antigen in fewer doses than if the immunogen were administered alone. Examples of adjuvants include Freund's adjuvant (complete or incomplete), Adjuvant 65 (containing peanut oil, mannide monooleate and aluminum monostearate) and mineral gels such as aluminum hydroxide, aluminum phosphate, or alum. Freund's adjuvant is no longer used in vaccine formulations for humans or for food animals because it contains nonmetabolizable mineral oil and is a potential carcinogen; however, the mineral gels are widely used in commercial veterinary vaccines.

## Claims

1. A polypeptide capable of eliciting an sIgA response in a mammal on administration to said mammal which comprises the amino acid sequence Ser-Lys-Gln-Asp-Ile-Gly-Ala-Leu-Lys-Gln-Glu-Leu-Ala-Lys-Lys-Asp-Glu-Gly-Asn-Lys or the amino acid sequence Ser-Lys-Gln-Asp-Ile-Gly-Ala-Leu-Lys-Gln-Glu-Leu-Ala-Lys-Lys-Asp-Glu-Gly-Asn-Lys which has at least one amino acid substitution that does not alter the conformation of the polypeptide.

2. A polypeptide capable of eliciting an sIgA response in a mammal on administration to said mammal which comprises the amino acid sequence Leu-Asp-Ala-Ser-Arg-Glu-Ala-Lys-Lys-Gln-Val-Glu-Lys-Asp-Leu-Ala-Asn-Leu-Thr-Ala-Glu-Leu or the amino acid sequence Leu-Asp-Ala-Ser-Arg-Glu-Ala-Lys-Lys-Gln-Val-Glu-Lys-Asp-Leu-Ala-Asn-Leu-Thr-Ala-Glu-Leu which has at least one amino acid substitution that does not alter the conformation of the polypeptide.

3. A polypeptide capable of eliciting an sIgA response in a mammal on administration to said mammal which comprises the amino acid sequence Glu-Lys-Gln-Ile-Ser-Asp-Ala-Ser-Arg-Gln or the amino acid sequence Glu-Lys-Gln-Ile-Ser-Asp-Ala-Ser-Arg-Gln which has at least one amino acid substitution that does not alter the conformation of the polypeptide.

4. An antigen conjugate capable of eliciting an sIgA response in a mammal on administration to said mammal which comprises a linkable carrier covalently linked to a polypeptide of any of claims 1 to 3.

5. An antigen conjugate according to claim 4 wherein the carrier is a natural protein carrier.

6. An antigen conjugate according to any one of claims 4 and 5 wherein the carrier is cholera toxin B.

7. Use of a polypeptide of any one of claims 1 to 3 or an antigen conjugate of any one of claims 4 to 6 for preparing a pharmaceutical preparation for controlling streptococcal infections.

8. Use according to claim 7 wherein the pharmaceutical preparation is for intranasal administration.

9. Use according to claim 7 wherein the pharmaceutical preparation is for oral administration.

10. A vaccine which comprises a biologically acceptable diluent and a sIgA stimulating amount of a polypeptide of any one of claims 1 to 3.

## Patentansprüche

1. Polypeptid mit der Fähigkeit, in einem Säuger eine sIgA-Antwort auszulösen, wenn es diesem Säuger verabreicht wird, das die Aminosäuresequenz Ser-Lys-Gln-Asp-Ile-Gly-Ala-Leu-Lys-Gln-Glu-Leu-Ala-Lys-Lys-Asp-Glu-Gly-Asn-Lys oder die Aminosäuresequenz Ser-Lys-Gln-Asp-Ile-Gly-Ala-Leu-Lys-Gln-Glu-Leu-Ala-Lys-Lys-Asp-Glu-Gly-Asn-Lys mit wenigstens einer Aminosäuresubstitution, die die Konformation des Polypeptids nicht verändert, umfaßt.

2. Polypeptid mit der Fähigkeit, in einem Säuger eine sIgA-Antwort auszulösen, wenn es diesem Säuger verabreicht wird, das die Aminosäuresequenz Leu-Asp-Ala-Ser-Arg-Glu-Ala-Lys-Lys-Gln-Val-Glu-Lys-Asp-Leu-Ala-Asn-Leu-Thr-Ala-Glu-Leu oder die Aminosäuresequenz Leu-Asp-Ala-Ser-Arg-Glu-Ala-Lys-Lys-Gln-Val-Glu-Lys-Asp-Leu-Ala-Asn-Leu-Thr-Ala-Glu-Leu mit wenigstens einer Aminosäuresubstitution, die die Konformation des Polypeptids nicht verändert, umfaßt.

3. Polypeptid mit der Fähigkeit, in einem Säuger eine sIgA-Antwort auszulösen, wenn es diesem Säuger verabreicht wird, das die Aminosäuresequenz Glu-Lys-Gln-Ile-Ser-Asp-Ala-Ser-Arg-Gln oder die Aminosäuresequenz Glu-Lys-Gln-Ile-Ser-Asp-Ala-Ser-Arg-Gln mit Wenigstens einer Aminosäuresubstitution, die die Konformation des Polyoeptids nicht verändert, umfaßt.

4. Konjugiertes Antigen mit der Fähigkeit, in einem Säuger eine sIgA-Antwort auszulösen, wenn es diesem Säuger verabreicht wird, das einen koppelbaren Carrier umfaßt, der kovalent an ein Polypeptid nach irgendeinem der Ansprüche 1 bis 3 gebunden ist.

5. Konjugiertes Antigen nach Anspruch 4, worin der Carrier ein natürlicher Proteincarrier ist.

6. Konjugiertes Antigen nach irgendeinem der Ansprüche 4 und 5, worin der Carrier Choleratoxin B ist.

7. Verwendung eines Polypeptids nach irgendeinem der Ansprüche 1 bis 3 oder eines konjugierten Antigens nach irgendeinem der Ansprüche 4 bis 6 zur Herstellung eines Arzneimittels zur Bekämpfung von Streptococceninfektionen.

8. Verwendung nach Anspruch 7, worin das Arzneimittel zur intranasalen Verabreichung ist.

9. Verwendung nach Anspruch 7, worin das Arzneimittel zur oralen Verabreichung ist.

10. Impfstoff, der ein biologisch annehmbares Diluens und eine sIgA-stimulierende Menge eines Polypeptids nach irgendeinem der Ansprüche 1 bis 3 umfaßt.

## Revendications

1. Polypeptide capable d'induire une réponse sIgA chez un mammifère lorsqu'il est administré audit mammifère, et qui comprend la séquence d'amino-acides Ser-Lys-Gln-Asp-Ile-Gly-Ala-Leu-Lys-Gln-Glu-Leu-Ala-Lys-Lys-Asp-Glu-Gly-Asn-Lys ou la séquence d'amino-acides Ser-Lys-Gln-Asp-Ile-Gly-Ala-Leu-Lys-Gln-Glu-Leu-Ala-Lys-Lys-Asp-Glu-Gly-Asn-Lys qui a au moins une substitution sur un amino-acide ne modifiant pas la conformation du polypeptide.

2. Polypeptide capable d'induire une réponse sIgA chez un mammifère lorsqu'il est administré audit mammifère, et qui comprend la séquence d'amino-acides Leu-Asp-Ala-Ser-Arg-Glu-Ala-Lys-Lys-Gln-Val-Glu-Lys-Asp-Leu-Ala-Asn-Leu-Thr-Ala-Glu-Leu ou la séquence d'amino-acides Leu-Asp-Ala-Ser-Arg-Glu-Ala-Lys-Lys-Gln-Val-Glu-Lys-Asp-Leu-Ala-Asn-Leu-Thr-Ala-Glu-Leu qui a au moins une substitution sur un amino-acide ne modifiant pas la conformation du polypeptide.

3. Polypeptide capable d'induire une réponse sIgA chez un mammifère lorsqu'il est administré audit mammifère, et qui comprend la séquence d'amino-acides Glu-Lys-Gln-Ile-Ser-Asp-Ala-Ser-Arg-Gln ou la séquence d'amino-acides Glu-Lys-Gln-Ile-Ser-Asp-Ala-Ser-Arg-Gln qui a au moins une substitution sur un amino-acide ne modifiant pas la conformation du polypeptide.

4. Conjugué antigène capable d'induire une réponse sIgA chez un mammifère lors de son administration audit mammifère qui comprend un support liable lié de manière covalente à un polypeptide selon l'une des revendications 1 à 3.

5. Conjugué antigène selon la revendication 4, dans lequel le support est un support protéinique naturel.

6. Conjugué antigène selon l'une des revendications et 5, dans lequel le support est la toxine B du choléra.

7. Utilisation d'un polypeptide selon l'une quelconque des revendications 1 à 3 ou d'un conjugué antigène selon l'une quelconque des revendications 4 à 6 pour préparer une préparation pharmaceutique en vue de la maîtrise d'infections à streptocoques.

8. Utilisation selon la revendication 7, dans laquelle la préparation pharmaceutique est destinée à une administration intra-nasale.

9. Utilisation selon la revendication 7, dans laquelle la préparation pharmaceutique est destinée à une administration orale.

10. Vaccin qui comprend un diluant acceptable sur le plan biologique et une quantité, efficace à stimuler le sIgA d'un polypeptide selon l'une quelconque des revendications 1 à 3.
